# EUROPEAN PATENT APPLICATION

(11) **EP 1 857 130 A1**
(43) Date of publication of application: **21.11.2007**
(21) Application number: 07108293.7
(22) Date of filing: 15.05.2007
(51) Int. Cl.: A61M 5/145, A61M 5/28, A61M 5/315

(54) **Drug delivery device**

(30) Priority: 17.05.2006 US 435906
(71) Applicant: Alcon, Inc., 6331 Hünenberg (CH)
(72) Inventor: Dacquay, Bruno, Irvine, CA California 92688 (US); Zica, Michael, Costa Mesa, CA California 92626 (US)
(74) Representative: Hanna, Peter William Derek

(57) **Abstract**

A syringe-like device (10) is provided having a drug dispensing tip (12) connected to a motorized handpiece (14). The tip may be packaged pre-loaded with an amount of a liquid or phase transition drug or may be shipped empty and loaded with a liquid or phase transition drug by the user. The motorized handpiece contains suitable sensors and controllers (24) for precisely actuating a plunger (21) in the tip for the controlled delivery of the liquid or phase transition drug from a needle or cannula (16). The handpiece may contain an internal power source (26), such as a battery, or may be connected to an external power source (126) and/or control unit (124).

## Description

The present invention generally pertains to the delivery of ophthalmically acceptable pharmaceutically active agents to the back of the eye and more particularly to an apparatus for delivery of precise amounts of a liquid or phase transition drug under controlled back pressure and flow conditions.

### Background of the Invention

Age related macular degeneration (ARMD) is the leading cause of blindness in the elderly. It attacks the center of vision and blurs it, making reading, driving, and other detailed tasks difficult or impossible. Current estimates reveal that approximately forty percent of the population over age 75, and approximately twenty percent of the population over age 60, suffer from some degree of macular degeneration. In "wet" ARMD, the type that most often causes blindness, newly formed choroidal blood vessels (CNV) leak fluid and cause progressive damage to the retina. Two main methods of treatment are currently being performed, (a) photocoagulation and (b) the use of angiogenesis inhibitors.

Photocoagulation can be harmful to the retina and is impractical when the CNV is in proximity of the fovea. Furthermore, photocoagulation often results in recurrent CNV over time.

Angiogenesis inhibitors may be administered orally, by intraocular injections, by extraocular sub-Tennon injections, or by local implants. In the sub-Tenon injection method, a physician disposes the tip of a curved needle externally around the eye near the macula, and releases a precise amount of the drug. However, a concern with sub-Tenon delivery is that the drug must be administered slowly and under relatively low pressure so as to be retained in the tissue rather than leaking back out, around the needle.

Therefore, a need exists in the field of ophthalmology for an improved apparatus for controlled delivery of a drug to the human eye.

### Brief Summary of the Invention

The present invention improves upon the prior art by providing a syringe-like device having a drug dispensing tip connected to a motorized handpiece. The tip may be packaged pre-loaded with an amount of a liquid or phase transition drug or may be shipped empty and loaded with a liquid or phase transition drug by the user. The motorized handpiece contains suitable sensors and controllers for precisely actuating the tip for the controlled delivery of the liquid or phase transition drug in the tip. The handpiece may contain an internal power source, such as a battery, or may be connected to an external power source and/or control unit.

Accordingly, one objective of the present invention is to provide a syringe-like device having a drug dispensing tip.

Another objective of the present invention is to provide a device for the delivery of a drug to the human eye and having a drug dispensing tip actuated by a motorized handpiece.

Yet another objective of the present invention is to provide a drug delivery device wherein the flow rate of the drug being delivered can be controlled.

Yet another objective of the present invention is to provide a drug delivery device wherein the amount of the drug being delivered can be controlled.

These and other advantages and objectives of the present invention will become apparent from the detailed description and claims that follow.

### Brief Description of the Drawings

FIG. 1 is an enlarged, schematical view of a first embodiment of the drug deliver device of the present invention.
FIG. 2 is an enlarged, schematical view of a second embodiment of the drug deliver device of the present invention.

### Detailed Description of the Invention

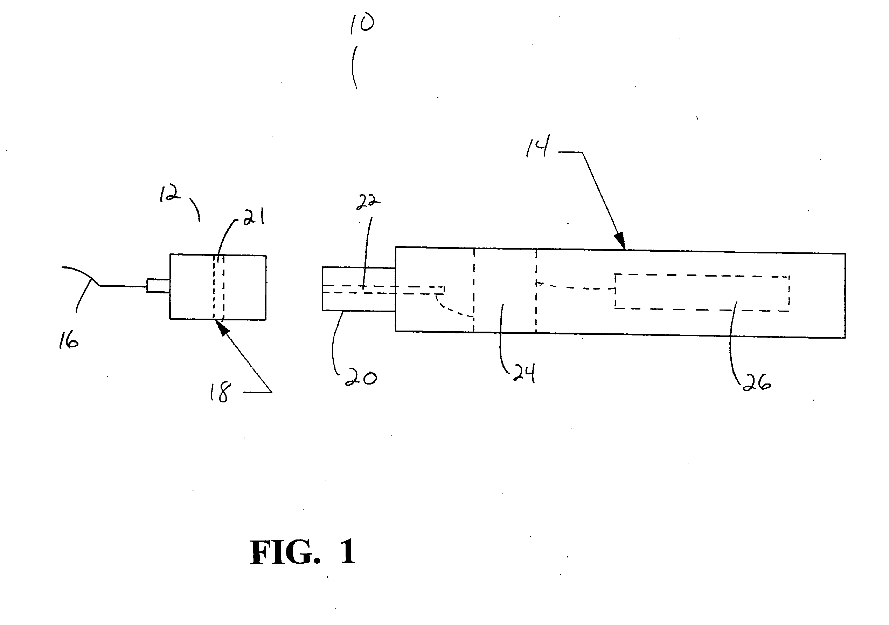

As best seen in FIG. 1, drug delivery device 10 of the present invention generally comprises tip 12 and handpiece 14. Tip 12 generally contains delivery cannula or needle 16 and body 18. Needle 16 may be any suitably sized needle of conventional construction. Body 18 generally is of a construction similar to a standard hypodermic syringe, *e.g*., cylindrical and tubular, made from glass, metal or a suitable plastic. Body 18 is sized and shaped to hold a suitable amount of a liquid or phase transition drug. Handpiece 14 generally is sized and shape similar to body 18, *e.g*., cylindrical, and has distal end 20 adapted to allow tip 12 to be mechanically connected to handpiece 14. Handpiece 14 contains drive mechanism 22, with associated driver electronics 24, to push plunger 21 in body 18 to force the liquid or phase transition drug contained in body 18 out needle 16. Suitable drive mechanisms include, for example, piezoelectric linear motors, such motors being well-known in the art (as described in U.S. Patent No. 6,940,209 (Henderson)), and available from sources such as New Scale Technologies, Inc., Victor, New York. In the embodiment illustrated in FIG. 1, power for driver 22 and control electronics 24 is provided from power source 26, such as a battery, located internal to handpiece 14. Such a construction allows for very precise and controlled movement of plunger 21. In addition, electronics 24 can provide a feedback mechanism that senses the back pressure being exerted against the expression of the drug out of needle 16 and make appropriate adjustments to the speed and direction of motor 22.

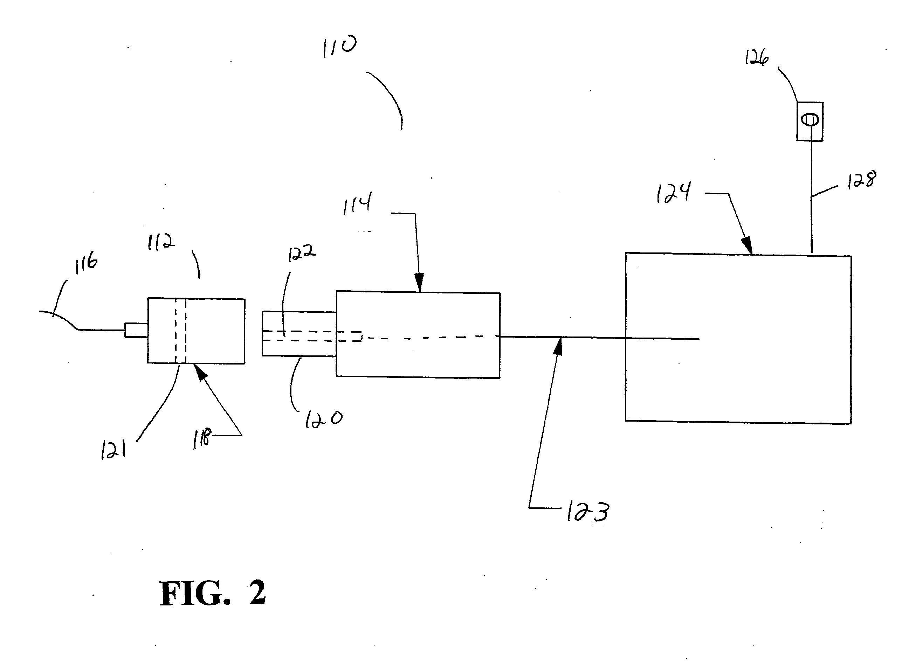

As best seen in FIG. 2, drug delivery device 110 of the present invention may also generally comprise tip 112 and handpiece 114. Tip 112 generally contains delivery cannula or needle 116 and body 118. Needle 116 may be any suitably sized needle of conventional construction. Body 118 is generally is of construction similar to a standard hypodermic syringe, *e.g.,* cylindrical and tubular, made from glass, metal or a suitable plastic. Body 118 is sized and shaped to hold a suitable amount of a liquid or phase transition drug. Handpiece 114 generally is sized and shaped similar to body 118, *e.g*., cylindrical, and has distal end 120 adapted so allow tip 112 to be mechanically connected to handpiece 114. Handpiece 114 contains drive mechanism 122, with associated driver electronics 124, to push plunger 121 in body 118 to force the liquid or phase transition drug contained in body 118 out needle 116. In the embodiment illustrated in FIG. 2, control electronics 124 are located external to handpiece 114 and power and control signals are provided to drive mechanism 122 through cable 123. Power for driver 122 and electronics 124 is provided from external source 126 through electrical cable 128. Such a construction allows for a smaller handpiece 114.

This description is given for purposes of illustration and explanation. It will be apparent to those skilled in the relevant art that changes and modifications may be made to the invention described above without departing from its scope or spirit.

## Claims

1. A drug delivery device (10,110), comprising:
a) a handpiece (14,114);
b) a tip (12,112) having a body (18,118), the body being adapted to be removably attached to the handpiece at one end;
c) a cannula (16,116) generally located on the body at an opposite end;
d) a plunger (21,121) located in the tip body; and
e) a drive mechanism (22,122) located in the handpiece (14,114), the drive mechanism being adapted to provide movement of the plunger when the tip is attached to the handpiece.

2. The drug delivery device of claim 1, wherein the drive mechanism (22) comprises a piezoelectric linear motor.

3. The drug delivery device of claim 1, wherein the handpiece (14) further comprises an internal power source (26) for the drive mechanism (22).

4. The drug delivery device of claim 1, wherein the handpiece (14) further comprises internal control electronics (24) for the drive mechanism (22).

5. The drug delivery device of claim 1, wherein the drive mechanism (122) is adapted to provide movement of the plunger (121) by means of external control electronics (124) for the drive mechanism.

6. The drug delivery device of claim 1, wherein the drive mechanism (122) is adapted to provide movement of the plunger (121) by means of an external power source (126) for the drive mechanism.

7. The drug delivery device of claim 4 or claim 5, wherein the control electronics (24,124) are adapted to provide a feedback mechanism that senses the back pressure being exerted against the expression of the drug out of the cannula (16,116) and makes appropriate adjustments to the speed and direction of the drive mechanism (22,122).
